Europäisches Patentamt

European Patent Office

Office européen des brevets

(11) Publication number: **0 359 425**
**A1**

(12) **EUROPEAN PATENT APPLICATION**

(21) Application number: 89308634.8

(22) Date of filing: 24.08.89

(51) Int. Cl.5: **C12N 15/55 , C12N 15/81 ,**
**C12N 9/18 , C12N 1/18 ,**
**//(C12N1/18,C12R1:865)**

The applicant has filed a statement in accordance with Rule 28 (4) EPC (issue of a sample only to an expert). Accession number(s) of the deposit(s): FERM BP-313

(30) Priority: 29.08.88 JP 214666/88

(43) Date of publication of application:
21.03.90 Bulletin 90/12

(84) Designated Contracting States:
AT BE CH DE ES FR GB GR IT LI LU NL SE

(71) Applicant: SHIONOGI SEIYAKU KABUSHIKI
KAISHA trading under the name of
SHIONOGI & CO. LTD.
3-1-8, Dosho-machi Chuo-ku
Osaka-shi Osaka(JP)

(72) Inventor: Tamaki, Mikio
1-2446-6, Sakinaka-machi
Nara-shi Nara-ken(JP)
Inventor: Takimoto, Noriko
6-2-1-304, Motoyamaminami-machi
Higashinada-ku
Kobe-shi Hyogo-ken(JP)
Inventor: Nakamura, Etsuo
4573-41, Karato Arino-cho Kita-ku
Kobe-shi Hyogo-ken(JP)
Inventor: Teraoka, Hiroshi
2-47-10, Takakuradai
Sakai-shi Osaka(JP)
Inventor: Ogawa, Michio
1-15-7, Uenozaka
Toyonaka-shi Osaka(JP)
Inventor: Matsubara, Kenichi
3-18-1-804, Yamadahigashi
Suita-shi Osaka(JP)

(74) Representative: Nash, David Allan et al
Haseltine Lake & Co. 28 Southampton
Buildings Chancery Lane
London WC2A 1AT(GB)

(54) A method for producing human pancreatic phospholipase A2.

(57) A method for producing human pancreatic phospholipase A2 is provided, that comprises the steps of, introducing a plasmid capable of expressing human pancreatic phospholipase A2 into yeast cells; culturing said yeast cells in a culture medium to induce the secretion of human pancreatic phospholipase A2; and recovering the secreted human pancreatic phospholipase A2 from the culture medium.

## A METHOD FOR PRODUCING HUMAN PANCREATIC PHOSPHOLIPASE A₂

This invention relates to a method for the efficient production of human phospholipase $A_2$ by the use of recombinant DNA techniques.

Phospholipase $A_2$ (EC 3.1.1.4) is a phospholipolytic enzyme which catalyzes the hydrolysis of the 2-acyl ester bond of 3-sn-phosphoglycerides, and is known to be present in mammalian pancreatic tissues as well as in various snake venoms. Pancreatic phospholipase $A_2$ is one of the digestive enzymes secreted in the pancreatic juice, and ordinarily exists as a precursor which is subsequently converted into the active form through hydrolysis by proteolytic enzymes such as trypsin. Phospholipase $A_2$ is also known to be widely distributed as an intracellular enzyme, principally as a membrane-bound enzyme, in mammals and microorganisms.

The phospholipase $A_2$ present in pancreatic tissue or in snake venoms, as well as phospholipase C (known to originate in microorganisms such as Clostridium welchii and Bacillus cereus) is indispensable for research on biological membranes, particularly the structural analysis of the phospholipids which are important membrane components. Furthermore, pancreatic phospholipase $A_2$ is known to be released into the bloodstream in various pancreatic disorders, causing elevations of blood levels of the enzyme, and therefore constituting a marker for the diagnosis of pancreatic disease. In order to employ phospholipase $A_2$ in the above-mentioned fields of research and in clinical diagnosis, adequate quantities of the enzyme are necessary. However, since human pancreatic phospholipase $A_2$ has hitherto been isolated and purified from human pancreas or pancreatic juice, large quantities of the phospholipase $A_2$ have not been obtained. Thus, there exists a vital need for the development of an effective method of producing large quantities of human phospholipase $A_2$.

In recent years, recombinant DNA techniques have come into increasingly wider use as a means for the mass production of specifically desired proteins. Examples of procedures for producing phospholipase $A_2$ in host organisms by recombinant DNA techniques include that reported by Tanaka et al. Gene, 64, 257 (1988), which effects secretory expression of bovine pancreatic phospholipase $A_2$ by yeast cells. According to this report, a DNA base sequence encoding bovine pancreatic phospholipase $A_2$ proenzyme is designed so as to use as much yeast optimal codons as possible, and this is synthesized from 22 oligomers with an average 42 mer chain length. The said DNA base sequence encoding the phospholipase $A_2$ proenzyme is prepared by appending a canine phospholipase $A_2$ signal sequence to the gene encoding the mature protein of bovine pancreatic phospholipase $A_2$. The said DNA base sequence is ligated downstream to the yeast PHO5 (acid phosphatase) promoter, inserted into the yeast-E. coli shuttle vector pAT405, and finally expressed in yeast, moreover, the secretion into the yeast culture medium of bovine pancreatic phospholipase $A_2$ almost identical with the natural substance has been verified.

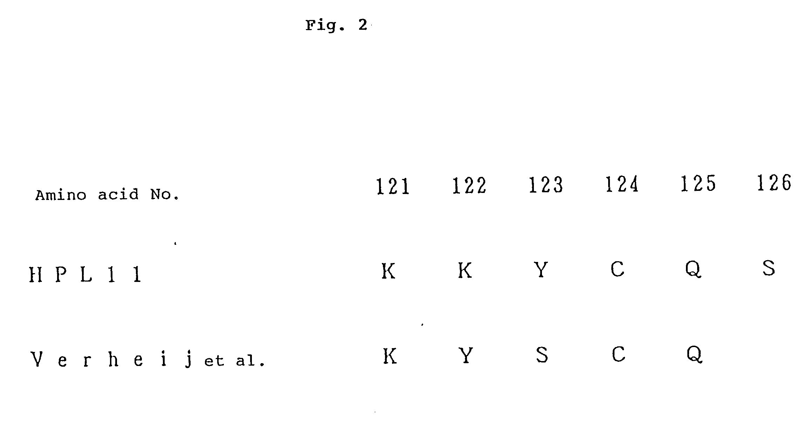

The amino acid sequence of human pancreatic phospholipase $A_2$ has been determined by Verheij et al. (Biochim. Biophys. Acta, 747 (1983), 93-99), using the techniques of protein chemistry. Furthermore, the DNA base sequence encoding human pancreatic phospholipase $A_2$ protein originating in the human lung has been determined by Seilhamer et al. (DNA, 5 (6) (1986), 519-527), and an amino acid sequence has been deduced from this base sequence. However, comparing the amino acid sequences established by Verheij with that deduced from the DNA base sequence determined by Seilhamer, certain differences in the 5 amino acids at the C terminus are observed (Figure 2). Moreover, the number of amino acid residues in the former sequence is 126, as compared with 125 amino acid residues in the latter sequence. The reason for this discrepancy is not clear, but may be the presence of phospholipase $A_2$ isozymes in the individuals from which the phospholipase $A_2$ analytes were obtained, or possibly an error in the amino acid analysis.

Thus, various research has been performed in connection with human pancreatic phospholipase $A_2$, but up until now no examples of production of this human pancreatic phospholipase $A_2$ in host cells using recombinant DNA techniques have been announced.

The method for producing human pancreatic phospholipase $A_2$ of this invention, which overcomes the above-discussed and numerous other disadvantages and deficiencies of the prior art, comprises the steps of, introducing a plasmid capable of expressing human pancreatic phospholipase $A_2$ into yeast cells; culturing said yeast cells in a culture medium to induce the secretory expression of human pancreatic phospholipase $A_2$; and recovering the secreted human pancreatic phospholipase $A_2$ from the culture medium.

In a preferred embodiment, the plasmid is prepared by the following steps: isolating mRNA from human pancreas; synthesizing double-stranded cDNA from said mRNA; preparing a cDNA library in lambda phage from said cDNA; screening a cDNA clone, which have a gene encoding human pancreatic phospholipase $A_2$, from said cDNA library; and introducing the gene encoding human pancreatic phospholipase $A_2$

2

contained in said cDNA clone into an expression vector.

In a preferred embodiment, the plasmid capable of expressing human pancreatic phospholipase A₂ is pAM82 HuPLA₂.

In a preferred embodiment, the yeast cells carrying said plasmid is Saccharomyces cerevisiae pAM82-HuPLA₂/AH22.

Thus, the invention described herein makes possible the objectives of (1) providing a method for producing human pancreatic phospholipase A₂ using recombinant DNA techniques, by which recombinant human pancreatic phospholipase A₂ possessing the same amino acid sequence as natural human pancreatic phospholipase A₂ is produced in large quantities at low cost; and (2) providing a method for manufacturing recombinant human pancreatic phospholipase A₂ that is useful as a diagostic reagent for clinical testing in cases of pancreatitis and other pancreatic diseases, and useful as an agent for basic research on the metabolism of phospholipids that constitute important components of biological membranes.

This invention may be better understood and its numerous objects and advantages will become apparent to those skilled in the art by reference to the accompanying drawings as follows:

Figure 1 shows the DNA base sequence (obtained from clone HPL11) encoding human pancreatic phospholipase A₂ which is used in the present invention, together with the amino acid sequence corresponding to the said DNA base sequence.

Figure 2 shows the C terminal region of the amino acid sequence corresponding to the DNA base sequence (obtained from clone HPL11) encoding human pancreatic phospholipase A₂ which is used in the present invention, compared with the C terminal region of the amino acid sequence of human pancreatic phospholipase A₂ as determined by Verheij et al.

Figure 3 shows the amino acid sequence corresponding to the DNA base sequence (obtained from the cDNA clone HPL11) encoding mature human pancreatic phospholipase A₂ used in the present invention, compared with the amino acid sequence of canine pancreatic phospholipase A₂.

Figure 4 is a schematic explanatory diagram indicating the procedure for construction of the expression plasmid pAM82 HuPLA₂ carrying the gene encoding human pancreatic phospholipase A₂ which is used in the present invention.

The gene encoding human pancreatic phospholipase A₂ which is employed in the present invention is prepared from human pancreas cells. The DNA base sequence of this gene has already been documented by Seilhamer et al. (supra), and can also be prepared by chemical synthesis. However, the carboxyl terminus of the amino acid sequence deduced from this base sequence is somewhat different from that of the amino acid sequence of human pancreatic phospholipase A₂ as reported by Verheij (supra). Consequently, in order to ensure that the authentic gene sequence is obtained, the gene must be directly prepared from human pancreas.

The production of human pancreatic phospholipase A₂ according to the method of the present invention can, for example, be implemented by the following sequence of processes, which will now be described in detail and in the proper order of execution in order to illustrate the method.

## (1) Preparation of human pancreatic cDNA library

The human cDNA library is prepared by the conventional method. That is, first, total cellular RNA is separated from human pancreas by the guanidine phenol/chloroform method (Gene, 28 (1984), 263-270), and this is repeatedly purified by oligo-dT cellulose column chromatography, thus obtaining mRNA with poly(A) segments, then double-stranded cDNA is produced from this mRNA. This production of double-stranded cDNA can be performed in accordance with the method of Gubler and Hoffman (Gene, 25 (1983), 263-269). In the double-stranded DNA so obtained, restriction enzyme recognition sites are formed by appending appropriate linkers, and then this DNA is inserted into a cloning vector at the corresponding cleavage site (e.g., if EcoR I linkers have been connected to the cDNA, then the cDNA would be inserted at the EcoR I cleavage site in the vector), resulting in a human pancreatic cDNA library. One cloning vector appropriate for this purpose is the phage vector lambda gt10, but the choice is not restricted to this variety, and in fact virtually any cloning vector known to those skilled in genetic engineering can be employed for this purpose.

## (2) Isolation of human pancreatic phospholipase A₂ cDNA clone

The human pancreatic cDNA library obtained in the manner described above is inserted into a suitable variety of host cell (i.e., phage plating cell) to effect transformation and cloning. For example, the E. coli NM514 strain or other Escherichia coli strains are suitable for this purpose. Prior to introduction of the phage, these bacteria are first grown in a shaking culture until the optical density measured with a spectrophotometer reaches 0.5, then the collected bacteria are suspended in a magnesium sulphate solution and stored at a low temperature. These phage plating cells are mixed with the phage liquid containing the aforementioned cDNA library, and E. coli transformants are obtained by incubating this mixture, which is then inoculated into a suitable culture medium, where the transformants form plaques. These are replica-plated onto nitrocellulose filters, then the bacteria are lysed and the DNA denatured by sodium hydroxide treatment, and the phage DNA in the plaques is bound to the filter. Next, using this filter, plaque hybridization is performed, thereby identifying the plaque containing the recombinant phage possessing the desired gene which codes for human pancreatic phospholipase $A_2$.

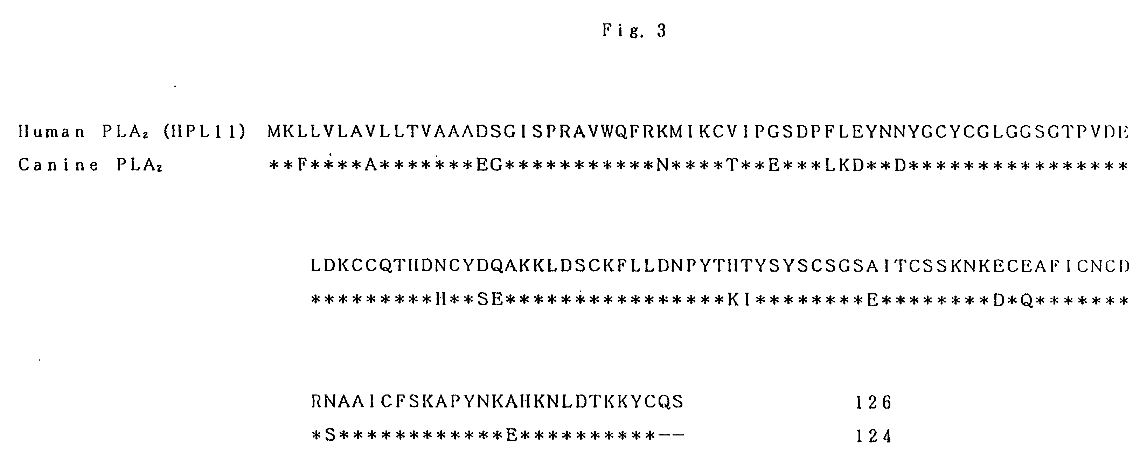

As a labelled probe, for example, a radio-actively labelled 419 bp fragment can be used, that is obtained by cleavage of the cDNA corresponding to canine pancreatic phospholipase $A_2$ (J. Biochem., 99, 733-739 (1986)) with the restriction enzyme Rsa I. Radioactive labelling can be conveniently performed, for example, with [$^{32}$P-$\alpha$]dCTP, using the Multiprime DNA Labeling System (Amersham). At the nucleotide level, the above-mentioned canine cDNA is 83% homologous to that of human pancreatic phospholipase $A_2$ (see Figure 3), and is therefore suitable as a probe for this purpose. Other varieties of animal DNA as well as synthetic DNA or RNA possessing base sequences which can hybridize with that of human pancreatic phospholipase $A_2$ can also be used as probes in this process.

Plaque hybridization is performed by first prehybridizing the filter, to which the aforesaid phage DNA is bound, with a buffer solution containing E. coli DNA, and next hybridizing in a solution containing the above-mentioned labelled probe ($2 \times 10^6$ cpm/filter). After this filter has been washed, autoradiographic analysis is used to screen for the clones of phage vectors which carry the desired gene encoding human pancreatic phospholipase $A_2$.

<center>(3) Analysis of cDNA clones</center>

In order to analyze the cDNA contained in the positive clones obtained as described in the preceding section 2, phage DNA is prepared from these clones. The preparation of phage DNA can be accomplished by proceeding in accordance with the protocol of Maniatis et al., as described in "Molecular Cloning, A Laboratory Manual, Cold Spring Harbor Laboratory Press, New York (1982)".

The phage DNA so obtained ( e.g., the varieties of phage DNA designated as HPL11 and HPL21 by the inventors) are cleaved with a restriction enzyme such as EcoR I, followed by phenol extraction and ethanol precipitation, and after gentle drying under reduced pressure, the precipitate is dissolved in water. Phenol extraction and ethanol precipitation are desirable after each enzyme treatment. The EcoR I digest obtained in this manner is then fractionated by agarose gel electrophoresis, whereupon ethidium bromide staining reveals the inserted DNA fragments contained in the aforementioned DNA phages HPL11 and HPL21 in the bands corresponding to approximately 600 bp and 675 bp, respectively.

Next, in order to determine the base sequences of the inserted DNA fragments, a restriction endonuclease map is composed. To accomplish this, first, the aforesaid EcoR I digest of phage DNA is subjected to polyacrylamide gel electrophoresis to separate the DNA fragments, which are then recovered from the polyacrylamide gel by, for example, electrophoretic adsorption onto a DEAE membrane filter (NA-45, Schlecher and Schuell) and elution from the filter. After phenol extraction and ethanol precipitation from the eluate, the DNA is digested with appropriate restriction enzymes, and the pattern of the cleavage sites is analyzed. The restriction endonucleases which can be employed for this purpose include, for example, Sma I, Pvu II, Stu I, Rsa I and Pst I, however, the choice is not confined to this list, and any suitable restriction enzymes among the numerous varieties employed by those skilled in genetic engineering can be selected and used. These restriction enzymes should, of course, be used under the conditions appropriate for each particular enzyme. This pattern analysis confirms that the phospholipase $A_2$ structural genes in the DNA insertion fragments contained in the aforesaid phage DNA HPL11 and HPL21 are completely identical.

(4) Base sequencing of cDNA clones of human pancreatic phospholipase $A_2$

The base sequence of the above-mentioned DNA insertion fragments can be determined by using, for

<center>4</center>

example, the DNA sequencing kit manufactured by Takara Shuzo, Ltd. Using this kit, a recombinant phage is obtained by inserting the DNA fragment to be investigated into an M13 phage vector, transforming a suitable host organism with this recombinant phage, and then performing screening. M13 phage vectors suitable for this purpose include M13mp10 and M13mp11. This analysis reveals that the DNA insertion fragments in the above-mentioned phage DNA HPL11 and HPL21 completely cover the required structural gene region for human pancreatic phospholipase $A_2$.

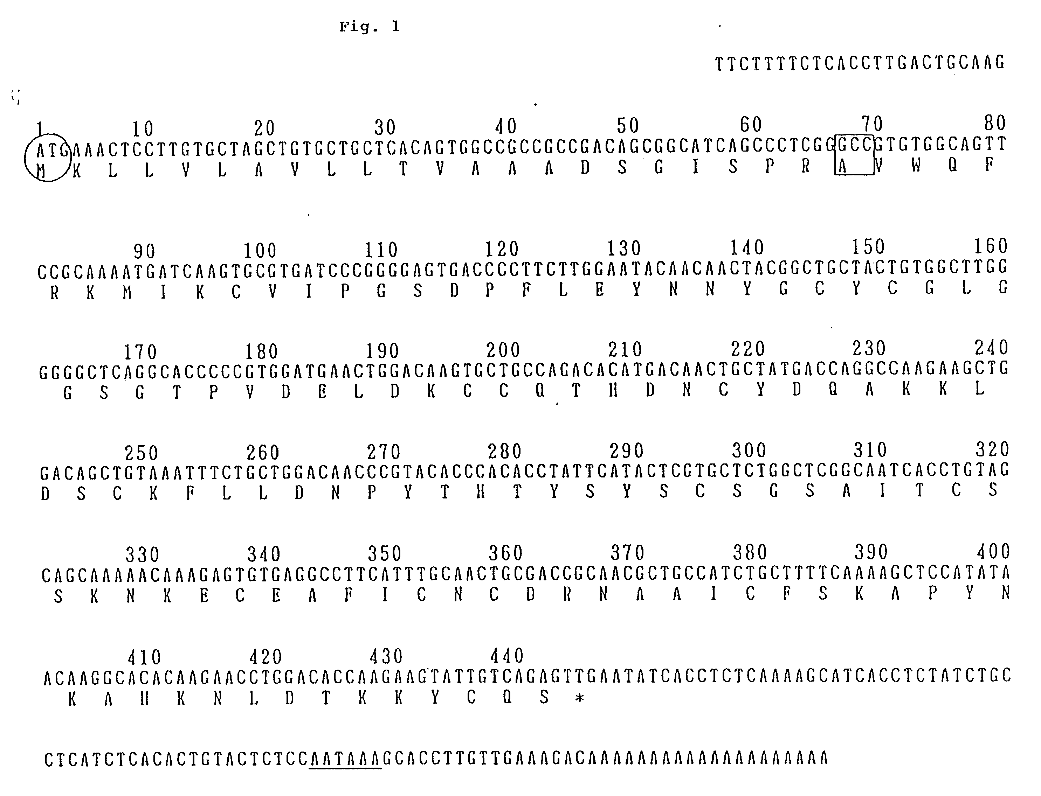

Figure 1 shows the base sequence of the DNA insertion fragment in HPL11 (upper sequence) as well as the amino acid sequence (lower sequence) deduced from this DNA base sequence. In this figure, the amino acid notation used in the lower sequence is as follows.

G: glycine (Gly)
A: alanine (Ala)
S: serine (Ser)
T: threonine (Thr)
C: cysteine (Cys)
N: asparagine (Asn)
Q: glutamine (Glu)
L: leucine (Leu)
I: isoleucine (Ile)
V: valine (Val)
M: methionine (Met)
F: phenylalanine (Phe)
Y: tyrosine (Tyr)
W: tryptophan (Trp)
P: proline (Pro)
D: aspartic acid (Asp)
E: glutamic acid (Glu)
H: histidine (His)
K: lysine (Lys)
R: arginine (Arg)

From Figure 1, it is seen that the base sequence of the said DNA insertion fragment contains a signal peptide sequence beginning with the translation start codon ATG coding for methionine, a propeptide sequence, the poly(A) signal AATAAA, and a poly(A) tail. The mature phospholipase $A_2$ polypeptide is composed of the 126 amino acid residues beginning with the A (alanine) residue at the 23rd position from the N terminus of the deduced amino acid sequence. The DNA base sequence encoding this phospholipase $A_2$ including the signal peptide and propeptide is completely identical with the human pancreatic phospholipase $A_2$ sequence reported by Seilhamer (supra).

The amino acid sequence of this human pancreatic phospholipase $A_2$ contains one more amino acid residue than that determined by Verheij et al. (supra) using protein biochemical techniques, and the differences between the two are confined to the C termini, which are compared in Figure 2, with the upper row of letters representing the C terminal region of the amino acid sequence obtained from HPL11, and the lower row representing that of the amino acid sequence determined by Verheij et al. Since the amino acid sequence deduced from the DNA sequence encoding the human pancreatic phospholipase $A_2$ obtained by the method of the present invention is completely identical with that of human pancreatic phospholipase $A_2$ originating in the human lung (Seilhamer et al., supra), the fact that .this sequence differs in the vicinity of the C terminus from the amino acid sequence reported by Verheij et al. suggests that an error may have occurred in the amino acid analysis performed by the latter authors.

Figure 3 shows the comparison between the amino acid sequence (upper row) deduced from the DNA base sequence obtained by the method of the present invention (i.e., that which encodes the human pancreatic phospholipase $A_2$ obtained from the DNA fragment inserted into phage DNA HPL11) and that of canine pancreatic phospholipase $A_2$ (lower row). The asterisks (*) in this figure indicate positions where the amino acid sequences represented by the upper and lower rows coincide. According to this comparison, human and canine pancreatic phospholipase $A_2$ are 82% homologous at the amino acid level and 83% homologous at the nucleotide level.

## (5) Construction of expression plasmids

Having obtained, in accordance with the procedure indicated in section 3 above, the phage DNA (HPL11 or HPL21) which includes DNA encoding human pancreatic phospholipase $A_2$, a portion encoding human pancreatic phospholipase $A_2$ is excised by means of restriction enzymes, the DNA fragments so obtained are then subjected to phenol-chloroform extraction and ethanol precipitation, and the precipitate is fractionated by agarose gel electrophoresis in the usual manner. These DNA fractions are recovered by electrophoretic adsorption onto a DEAE membrane filter followed by liquid elution, as indicated in section 3 above. The DNA fragments so obtained are then inserted into a suitable plasmid and cloned by introduction into host cells such as E. coli, etc. If this is done, for example, using the commercially available plasmid pGEM3, then the recombinant plasmid HuPLA$_2$/pGEM3 is obtained, that contains the desired DNA fragment.

Next, the desired DNA fragment is excised from this recombinant plasmid with restriction enzymes, and a phospholipase $A_2$ expression plasmid is then obtained by splicing the said DNA fragment into an appropriate plasmid vector. For example, by using T4 ligase to ligate

1) the approximately 750 bp fragment obtained by cleavage of the above-mentioned plasmid HuPLA$_2$/pGEM3 with Sal I and Pvu II and

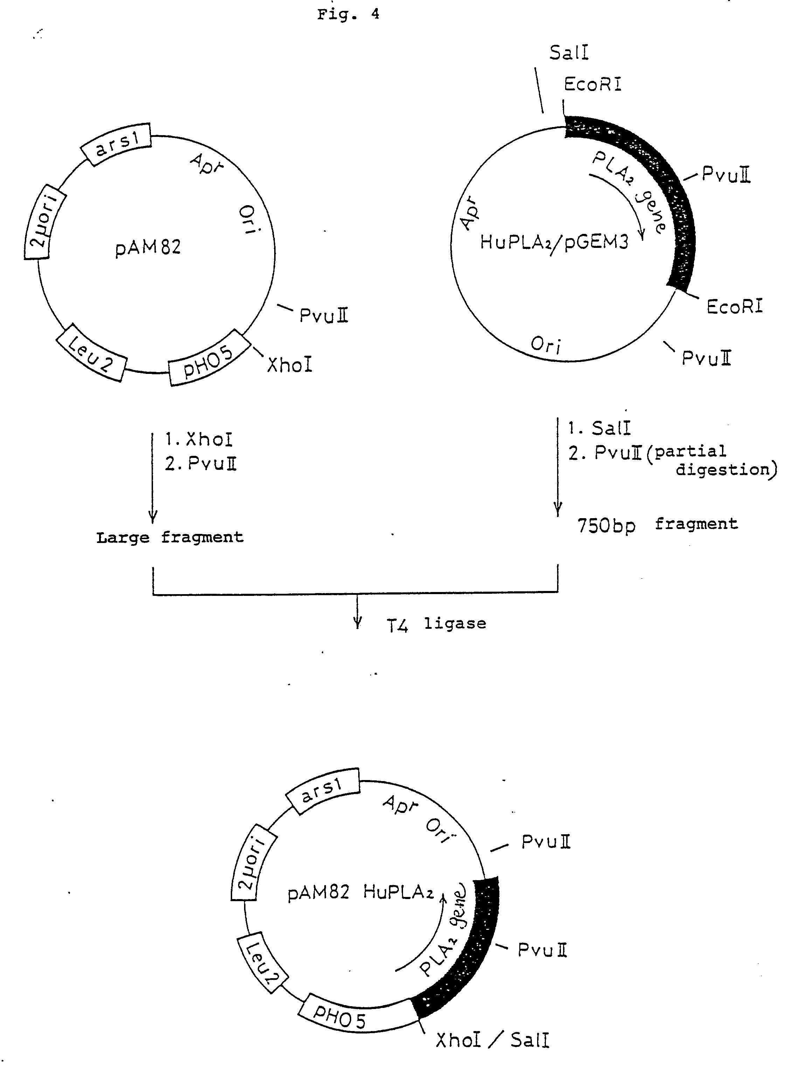

2) the large fragment obtained by cleavage of the plasmid pAM82 (described below) with Xho I and Pvu II, then the expression plasmid pAM82 HuPLA$_2$ is obtained. The basic procedure for construction of this expression plasmid pAM82 HuPLA$_2$ is schematically indicated in Figure 4. As the vector used in this construction, a shuttle vector which can replicate in both E. coli and Saccharomyces is most desirable. For example, the aforementioned plasmid pAM82 is suitable for this purpose. This plasmid pAM82 is disclosed in Japanese Patent Publication Nos. 61-55950 and 61-55951. A yeast strain carrying the plasmid pAM82 (Saccharomyces cerevisiae AH22/pAM82) has been deposited with the Fermentation Research Institute, Ibaraki, Japan, under the Accession Number FERM BP-313, and is available from that Institute. This plasmid vector contains both yeast and E. coli genes, and in particular the yeast repressible acid phosphatase gene (PHO5) and promoter. Any other shuttle vector among those generally known to those skilled in genetic engineering can also be used, provided that they can carry the aforementioned gene encoding human pancreatic phospholipase $A_2$ and replicate in both E. coli and yeast.

The expression plasmid constructed from the above-mentioned components 1) and 2) can been introduced into a suitable host such as E. coli (e.g., E. coli K12 strain C600), screened by drug resistance, and then subjected to analysis of restriction enzyme cleavage patterns to verify that the desired DNA fragment has been inserted at the correct position and with the proper orientation relative to the PHO5 promoter.

## (6) Expression of human pancreatic phospholipase $A_2$ in yeast

Yeast cells are transformed by the introduction of the expression plasmid pAM82 HuPLA$_2$ constructed in the manner described in section 5 above to obtain transformants that produce human pancreatic phospholipase $A_2$. The AH22 yeast strain is suitable for this purpose, but the choice is not limited to this strain, and in fact any type of yeast which is capable of expressing human pancreatic phospholipase $A_2$ upon the introduction of the aforementioned expression plasmid can also be used for the present purpose. Transformation can be effected in accordance with the method of Kimura et al. (J. Bacteriol. 153 (1983), 163-168). The recombinant clone (i.e., pAM82-HuPLA$_2$/AH22) carrying the desired expression plasmid can be obtained by selection with respect to leucine prototrophy on an agar plate containing histidine. This clone, pAM82-HuPLA$_2$/AH22, possesses the PHO5 promoter, and hence expression can readily be initiated or terminated by the presence or absence of inorganic phosphate in the culture medium (Nakao et al., Molec. Cell. Biol. 6 (1986), 2613-2623). For example, in accordance with the method of Miyanohara et al. (Proc. Natl. Acad. Sci. U.S.A. 80 (1983), 1-5), culture media containing and lacking phosphate can be prepared, and expression can be induced under hypophosphatic conditions. Since the expressed human pancreatic phospholipase $A_2$ is secreted, the produced phospholipase $A_2$ accumulates in the culture medium and is readily recovered by collecting the centrifugal supernatant fraction. The phospholipase $A_2$ activity of this supernatant fraction can be verified by assaying in accordance with the method of Okamoto et al. (J. Biochem. (Tokyo) 93 (1983), 1353), using a radiolabelled substrate (e.g., 1-palmitoyl-2[1-$^{14}$C]-linoleoylphosphatidylethanolamine). The human pancreatic phospholipase $A_2$ expressed by the yeast in this manner can be purified from the said yeast culture medium by an appropriate combination of various conventional chromatographic methods, such as gel filtration chromatography, ion exchange chromatography, affinity chromatography, etc.

Thus, by the method of the present invention, phospholipase $A_2$ possessing essentially the same amino acid sequence and activity as natural human pancreatic phospholipase $A_2$ can be obtained by recombinant DNA techniques.

Examples of the present invention will be described below.

## Example 1

### (1) Preparation of human pancreatic cDNA library

Human pancreatic tissue removed in a surgical operation was frozen in liquid nitrogen immediately after excision and stored at -70°C. Whole cell RNA was then separated from this pancreatic tissue by the guanidine phenol/chloroform method (Gene, 28 (1984), 263-270). Next, polyadenylated mRNA was purified from the whole-cell RNA by repeated oligo-dT cellulose column chromatography, then the corresponding double-stranded cDNA was prepared in accordance with the method of Gubler and Hoffman (Gene, 25 - (1983), 263-269). Then, EcoR I linkers were appended to the double-stranded cDNA so obtained, and a human pancreatic cDNA library was prepared by inserting these double strands into the phage cloning vector lambda gt10 at the EcoR I cleavage site of the vector.

### (2) Isolation of human pancreatic phospholipase $A_2$ cDNA clone

In order to prepare phage plating cells suitable for transfection with the aforesaid library, E. coli NM514 strain was inoculated into LB agar plates containing 1% trypton, 0.5% yeast extract, 0.5% sodium chloride and 1.5% agar, and the plates were incubated overnight at 37°C. Then, a single colony from this culture was transferred to 5 ml of LB medium containing 1% trypton, 0.5% yeast extract, 0.5% sodium chloride and 0.4% maltose, and this inoculated medium was shake-cultured overnight at 37°C. Next, 2 ml of this culture was added to 100 ml of the same LB medium, which was then shake-cultured at 37°C until the 600 nm optical density measured with a Shimadzu UV150-02 spectro-photometer reached 0.5. The culture so obtained was then centrifuged at 3,000 rpm for 10 minutes at a temperature of 4°C using Sorvall RC-5B, and then suspended in 10 ml of a pre-cooled 10 mM solution of magnesium sulphate. The phage plating cell suspension prepared in this manner was stored at 4°C.

Next, the phage solution containing the human pancreatic cDNA library obtained as indicated in section 1 above was diluted with a phage diluent solution containing 10 mM Tris HCl (pH 7.5), 10 mM sodium chloride and 0.1 mM EDTA, thus forming a solution with a phage concentration of $4 \times 10^4$/ml. A 0.1 ml aliquot of this diluted phage solution was then mixed with 0.1 ml of the aforesaid phage plating cell suspension, and after incubation at 37°C for 15 minutes, 4 ml of LB soft top agarose containing 1% trypton, 0.5% yeast extract, 0.5% sodium chloride, 0.25% magnesium sulphate and 0.72% agarose was added and the mixture was poured onto an LB agar plate. After standing at room temperature for 10 minutes, this plate was incubated at 37°C until plaques of diameter 1 to 1.5 mm had been formed, after which $4 \times 10^4$ plaques formed by the phage were transferred to 10 nitrocellulose filters, which were then immersed in an alkaline solution containing 0.5 M sodium hydroxide and 1.5 M sodium chloride for 1 minute. Next, these filters were immersed in a neutralizing solution containing 0.5 M Tris HCl (pH 7.5) and 1.5 M sodium chloride for 8 minutes and then in 3 x SSC (1 x SSC contains 150 mM sodium chloride and 15 mM sodium citrate) for 5 minutes. These filters were then air dried and dried in a vacuum oven at 80°C for 2 hours.

Next, the filters were prehybridized for 4 hours at 65°C in a mixture of 4 x SSC, 10 x Denhart's solution (Biochem. Biophys. Res. Comm. 23, 641-646 (1966)) and 200 μg/ml E. coli DNA, and then hybridized for 16 hours at 42°C in a mixture of 50% formamide, 4 x SSC, 10 x Denhart's solution, 200 μg/ml E. coli DNA and a $^{32}$P-probe ($2 \times 10^6$ cpm/filter) to complete plaque hybridization. The $^{32}$P-probe used was the 419 bp fragment obtained by cleaving canine pancreatic phospholipase $A_2$ cDNA (J. Biochem. 99 (1986), 733-739) with the restriction endonuclease Rsa I, which prior to use was labeled with [$^{32}$P-α] dCTP by means of the Multiprime DNA Labeling System (Amersham), thereby forming a DNA fragment with specific radioactivity $5 \times 10^8$ to $7 \times 10^8$ cpm/ μg. After hybridization, the filters were washed at room temperature with 4 x SSC and analyzed by autoradiography, which revealed 195 positive clones.

### (3) Analysis of cDNA clones

7

Two plaques on the medium were arbitrarily selected from among those corresponding to the 195 positive clones screened as indicated in section 2 above, aspirated with a Pasteur pipette and separately added to 1 ml portions of phage diluent, which were left standing overnight in an ice bath to extract the phage. Each extracted phage sample was then centrifuged at 10,000 rpm for 10 minutes at a temperature of 4°C and the supernatant was added to a tube containing 10μl chloroform, which was then stored at 4°C. The liquid containing the extracted phage was separated from the mixture, diluted with phage diluent, introduced into E. coli, and the number of phage contained in the sample was investigated by counting the number of plaques which appeared after incubation. Then, this phage solution containing $10^9$ phage was incubated at 37°C for 15 minutes together with the phage plating cells ($10^{11}$ cells) described in section 2 above. This mixture was then added to 1 liter of LB medium containing 5 mM calcium chloride which had been warmed beforehand at 37°C, and this mixture was incubated at 37°C for 6 hours. To this was added 5 ml of chloroform, and after further incubation for 15 minutes, the sample was immediately ice-cooled and centrifuged at 5,000 rpm for 15 minutes. Then, to the supernatant so obtained, RNase and DNase were added in amounts such that the concentration of each would be 1 μg/ml, and this mixture was maintained at 37°C for 1 hour. Next, at room temperature, 60 g of sodium chloride per liter of the supernatant was gradually added while stirring, and completely dissolved in the supernatant. Then, to the solution so obtained, 100 g of polyethylene glycol #6000 per liter was gradually added while stirring and completely dissolved, after which the mixture was left standing in an ice bath for more than one hour. Next, the mixture was centrifuged at 10,000 rpm for 20 minutes at 4°C, and the precipitate so obtained was suspended in SM buffer (50 mM Tris HCl (pH 7.5), 8 mM magnesium sulphate, 100 mM sodium chloride and 0.01% gelatin), forming a total volume of 9 ml. After adding and completely dissolving 6.75 g of cesium chloride in this suspension, the phage was recovered by density gradient centrifugation at 28,000 rpm for 24 hours at a temperature of 15°C using Beckman ultracentrifuge (Rotor-50Ti). After this phage solution had been dialyzed against an SM buffer solution, SDS (i.e., sodium lauryl sulphate) and EDTA (pH 8.0) were added so as to constitute concentrations of 0.2% and 10 mM, respectively, and the sample was maintained at 65°C for 10 minμg/ml of proteinase K was added, and the mixture was incubated at 37°C for 1 hour, after which an equal volume of a mixed phenol solution (phenol:chloroform:isoamyl alcohol in the proportions 25:24:1) was added, and the obtained mixture was mixed by inversion 30 times to effect phenol extraction of the DNA. The extracted DNA was centrifuged at 10,000 rpm for 20 minutes at 20°C, and the upper layer was recovered with a pipette. This phenol extraction procedure was repeated twice, and finally the DNA was recovered by collecting the supernatant. This supernatant was then dialyzed against TE buffer (10 mM Tris HCl (pH 8.0) and 1 mM EDTA) and the phage DNA was recovered from the residual liquid. The DNAs obtained from the two plaques mentioned above were designated as HPL11 and HPL21, respectively.

Next, 10 μg of each of the aforementioned phage was treated for 60 minutes at 37°C with 30 units of EcoR I in 100 μl of an EcoR I buffer solution (100 mM Tris HCl (pH 7.5), 7 mM magnesium chloride, 50 mM sodium chloride and 7 mM 2-mercaptoethanol). After the completion of the reaction, phenol extraction was performed in the manner indicated above, then ethanol precipitation was effected by adding a 1/10 volume of 3 M sodium acetate (pH 5.3) and 2.5 volumes of ethanol, and after gentle vacuum drying the precipitate was dissolved in water. These phenol and ethanol precipitation operations were performed after each enzyme treatment in all the processes described below. The EcoR I digest obtained as indicated above was fractionated by 1% agarose gel electrophoresis and ethidium bromide staining revealed the cDNA insertion fragments of HPL11 and HPL21 at bands corresponding to approximately 600 bp and 675 bp, respectively.

Next, in order to determine the DNA base sequence, the aforementioned EcoR I digest of phage DNA was subjected to 3.5% polyacrylamide gel electrophoresis. After ethidium bromide staining, the electrophoretic positions of the DNA fragments were revealed by ultraviolet (UV) irradiation, and the desired DNA fragment was electrophoretically recovered onto a DEAE membrane filter, NA-45 (Schleicher and Schuell). After gentle washing with a solution containing 0.1 M sodium chloride, 10 mM Tris HCl (pH 8.0) and 1 mM EDTA, the DNA was eluted from this filter by treatment with a solution containing 1 M sodium chloride, 10 mM Tris HC1 (pH 8.0) and 1 mM EDTA at 65°C for 30 minutes, then phenol extraction and ethanol precipitation were performed as indicated above, and the precipitate so obtained was dissolved in water. The DNA in this aqueous solution was then digested with Sma I, Pvu II, Stu I, Rsa I, Pst I and other suitable restriction enzymes, which were employed under the appropriate conditions indicated in Table 1 below.

Table 1

| Restriction enzyme | Final concentration (mM) | | | | | |
|---|---|---|---|---|---|---|
| | Tris HCl buffer (mM) | pH | KCl (mM) | NaCl (mM) | MgCl (mM) | 2-Mercapto ethanol |
| Sma I | 10 | 8.0 | 20 | - | 7 | 7 |
| Pvu II | 10 | 7.5 | - | 60 | 7 | 7 |
| Stu I | 10 | 8.0 | - | 100 | 7 | 7 |
| Rsa I | 10 | 7.5 | - | 50 | 7 | 7 |
| Pst I | 20 | 7.5 | - | 100 | 7 | - |

Analysis of the pattern of cleavage sites of these various restriction enzymes confirmed that the aforementioned two varieties of phage DNA, HPL11 and HPL21, were completely identical.

(4) Base sequencing of cDNA clones for human pancreatic phospholipase $A_2$

The base sequence of the cDNA obtained as described above was determined with a DNA sequencing kit (purchased from Takara Shuzo), using M13mp10 and M13mp11 as cloning vectors. The results demonstrated that the proteins encoded by the DNA insertion fragments in the above-mentioned two varieties of phage DNA HPL11 and HPL21, were identical, and that the entire range of the structural gene for human pancreatic phospholipase $A_2$ was covered. Figure 1 shows the base sequence of the DNA insertion fragment contained in HPL11 as well as the amino acid sequence deduced on the basis of the said base sequence. This insertion fragment contains a base sequence beginning with the translation start codon ATG (coding for methionine) which encodes the signal peptide and a base sequence encoding propeptide as well as an AATAAA poly(A) signal and a poly(A) tail, indicating that the corresponding mature phospholipase $A_2$ polypeptide comprises the 126 amino acid residues beginning with the A (alanine) at the 23rd position from the N terminus of the deduced amino acid sequence.

(5) Construction of expression plasmid

First, 10 μg of the phage DNA HPL11 or HPL21 obtained as described in section 3 above was treated for 60 minutes with 80 units of EcoR I in a buffer solution (100 mM Tris HCl (pH 7.5), 7 mM magnesium chloride, 50 mM sodium chloride, 7 mM 2-mercaptoethanol and 0.01% bovine serum albumin) at 37° C. After completion of the reaction, phenol-chloroform extraction was performed, and next ethanol precipitation was effected by adding 1/10 volume of 3 M sodium acetate (pH 5.3) and 2.5 volumes of ethanol. After gentle vacuum drying, the precipitate so obtained was dissolved in water. This EcoR I digest was then fractionated by 1% agarose gel electrophoresis, the positions of the DNA fragments were revealed by ethidium bromide staining and ultraviolet irradiation, and the desired fragment was electrophoretically recovered from the gel with a DEAE membrane filter. After washing with a washing solution (0.15 M sodium chloride/TE), the DNA was eluted by treating the DEAE membrane filter in the eluent (1 M sodium chloride/TE) at 65° C for 30 minutes, and the desired DNA fragment was recovered from this eluent by ethanol precipitation.

On the other hand, 1 μg of the commercially available plasmid pGEM3 (purchased from Promega Biotech), which carries an ampicillin resistance gene, was cleaved with EcoR I and then treated for 90 minutes with 0.5 units of alkaline phosphatase in 30 μl of 1 M Tris HCl (pH 8.0) at 65° C.

The EcoR I fragments containing human pancreatic phospholipase $A_2$ which were obtained in the above manner, together with 0.16 μg of pGEM3 which had also been cleaved by EcoR I, were treated for 18 hours with 350 units of T4 DNA ligase in a buffer solution (66 mM Tris HCl (pH 7.6), 6.6 mM magnesium chloride, 0.4 mM ATP and 10 mM dithiothreitol) at 4° C to effect a ligation reaction.

Using this reaction mixture, E. coli K12 strain C600 cells were transformed, and the transformed bacteria were selected for ampicillin resistance on agar plates containing ampicillin. Several of the ampicillin-resistant colonies were then chosen arbitrarily, the plasmid DNA was isolated, and the presence and orientation of the desired DNA fragments were checked by restriction enzyme cleavage pattern analysis. The plasmid which was found by this analysis to contain the desired DNA fragment was designated as HuPLA$_2$/pGEM3, and the transformed bacteria containing this HuPLA$_2$/pGEM3 plasmid were selected.

Next, the aforementioned plasmid HuPLA$_2$/pGEM3 was cleaved with Sal I. Then, this Sal I-cleaved DNA was treated for 60 minutes with 3.6 units of Pvu II in a buffer solution (10 mM Tris HCl (pH 7.5), 7 mM magnesium chloride, 60 mM sodium chloride and 7 mM 2-mercaptoethanol) at 37oC. After treatment, this liquid was subjected to 1% agarose gel electrophoresis in the manner indicated above and the Sal I-Pvu II fragment (750 bp) containing the desired structural gene for human pancreatic phospholipase A$_2$ was recovered.

Next, in order to construct an expression plasmid, this Sal I-Pvu II fragment was inserted into the DNA of plasmid pAM82. This plasmid pAM82, which had been doubly cleaved beforehand with Xho I and Pvu II, was then treated with 350 units of T4 DNA ligase at 4°C in 12 μl of the above-mentioned T4 DNA ligase buffer solution for 18 hours. By this treatment, the Sal I cleavage site of the phospholipase A$_2$ gene was joined to the Xho I cleavage site of the plasmid pAM82, thereby constructing the expression plasmid pAM82 HuPLA$_2$ with an inserted Sal I-Pvu II fragment containing the structural gene for phospholipase A$_2$; a schematic diagram illustrating the construction of this expression plasmid is shown in Figure 4. E. coli K12 strain C600 was then transformed by introduction of this expression plasmid.

The transformed bacteria was selected with agar plates containing ampicillin, and several of the ampicillin-resistant colonies were chosen arbitrarily. Plasmid DNA was then isolated from these colonies and restriction enzyme cleavage pattern analysis was used to verify the presence in this DNA of an insertion fragment carrying the desired human pancreatic phospholipase A$_2$ structural genes as well as the correct orientation of this DNA insertion fragment with respect to the PHO5 promoter.

### (6) Expression of human pancreatic phospholipase A$_2$ in yeast

Using the expression plasmid pAM82 HuPLA$_2$ constructed as described in section 5 above, the yeast AH22 strain was transformed in accordance with the method of Kimura et al. (J. Bacteriol. 153 (1983), 163-168). The transformant was selected with agar plates containing histidine, and the leucine-prototrophic yeast so obtained (pAM82-HuPLA$_2$/AH22) was used for expression in the following manner.

The yeast clone (pAM82-HuPLA$_2$/AH22), which had been verified as possessing the desired expression plasmid, carries the PHO5 promoter, and therefore expression can be readily initiated or stopped by the presence or absence of inorganic phosphate in a medium (Nakao et al., Molec. Cell. Biol. 6 (1986), 2613-2623). That is, since this promoter is induced under hypophosphatic conditions, p$^+$ medium (i.e., medium containing inorganic phosphate) and p$^-$ medium (i.e., medium not containing inorganic phosphate) were prepared in accordance with the method of Miyanohara et al. (Proc. Natl. Acad. Sci. U.S.A. 80 (1983), 1-5), and the following experiment concerning expression of phospholipase A$_2$ was performed.

### Preparation of medium

P$^+$ medium, with added inorganic phosphate (containing 1.5 g/l potassium dihydrogenphosphate) and p$^-$ medium, without added inorganic phosphate (containing 1.5 g/l potassium chloride) were prepared from Burkholder's minimal medium. For this purpose, first, stock solutions containing the metallic components listed in Table 2 as well as a stock solution containing the vitamins listed in Table 3 used in the p$^+$ and p$^-$ media were prepared. Using these, stock solutions containing fourfold concentrations of the components used in the p$^+$ and p$^-$ media (Tables 4 and 5, respectively), and finally the p$^+$ and p$^-$ media themselves, were prepared.

Table 2

| Stock solutions containing metallic components[a] | |
|---|---|
| Boron, manganese, zinc and copper stock solution (50ml) | |
| Boric acid | 30 mg |
| Manganese sulphate heptahydrate | 50 mg |
| Zinc sulphate heptahydrate | 150 mg |
| Cupric sulphate pentahydrate | 20 mg |
| (The above quantities were dissolved in sterile water so as to form a final total volume of 50 ml). | |
| Iron stock solution (50ml) | |
| Ferric chloride hexahydrate | 125 mg |
| (dissolved in sterile water so as to form a final total volume of 50 ml) | |
| Molybdenum stock solution (50 ml) | |
| Sodium molybdenate dihydrate | 100 mg |
| (dissolved in sterile water so as to form a final total volume of 50 ml) | |

a) autoclave sterilization was not performed.

Table 3

| Vitamin stock solution[b] | |
|---|---|
| Vitamin B$_1$ | 20 mg |
| Pyridoxine | 20 mg |
| Nicotinic acid | 20 mg |
| Calcium pantothenate | 20 mg |
| Biotin | 0.2 mg |
| Inositol | 1 g |
| (The above quantities were dissolved in sterile water so as to form a final total volume of 100ml). | |

b) Sterilization was performed by filtration through a Millipore filter.

Table 4

| Fourfold concentrated stock solution for p$^+$ medium[c] | |
|---|---|
| Potassium dihydrogen phosphate         . | 6 g |
| Magnesium sulphate heptahydrate | 2 g |
| Calcium chloride dihydrate | 1.32 g |
| 0.05% potassium iodide | 0.8 ml |
| Stock solutions for metallic components (Table 2) | |
| Boron, manganese, zinc and copper Stock solution | 0.2 ml |
| Iron stock solution | 0.2 ml |
| Molybdenum stock solution | 0.2 ml |
| (The above quantities were added to sterile water so as to form a final total volume of 1 liter). | |

c) Autoclave sterilization was not performed.

Table 5

| Fourfold concentrated stock solution for p$^-$ medium[d] | |
|---|---|
| Potassium chloride | 6 g |
| Magnesium sulphate heptahydrate | 2 g |
| Calcium chloride dihydrate | 1.32 g |
| 0.05% potassium iodide | 0.8 ml |
| Stock solutions for metallic components (Table 2) | |
| Boron, manganese, zinc and copper Stock solution | 0.2 ml |
| Iron stock solution | 0.2 ml |
| Molybdenum stock solution | 0.2 ml |
| (The above quantities were added to sterile water so as to form a final total volume of 1 liter). | |

d) Autoclave sterilization was not performed.

250 ml of either the p$^+$ or p$^-$ medium, 1 ml of the vitamin stock solution, 20 g of glucose and 2 g of asparagine were combined with water to form a total volume of 1 liter, and after thorough mixing, 50 mg of histidine hydrochloride was added, and using 0.2 N sodium hydroxide, the p$^+$ and p$^-$ media were adjusted to pH 6.0 and pH 7.5, respectively. Then, the p$^+$ and p$^-$ media were autoclaved for 10 minutes at 120°C and 110°C, respectively.

Induction of phospholipase A$_2$ expression

The colonies grown on the aforementioned histidine-containing agar plates were transferred into 10 ml of p$^+$ medium and shake-cultured for two days and nights at 30°C, then 0.2 ml of the culture medium was added to 10 ml of p$^-$ medium containing 0.05 ml of 2 M Tris HCl (pH 7.2), and this mixture was shake-cultured at 30°C for 2-5 days.

Assay of phospholipase A$_2$ activity

The aforesaid culture in the p$^-$ medium was centrifuged for 5 minutes at 2,500 x g, and the resulting supernatant was stored as the secretory fraction. The phospholipase $A_2$ activity of this secretory fraction was assayed fundamentally by the method of Okamoto et al. (J. Biochem. (Tokyo) 93 (1983), 1353) as follows. First, the aforesaid secretory fraction was allowed to react with 1-palmitoyl-2[1-$^{14}$C]-linoleoylphosphatidylethanolamine (55,000 dpm, 50 nmol) in an Eppendorf tube for 30 minutes in the presence of 10 mM calcium chloride and 0.1% sodium deoxycholate. Then, 0.1 ml of a 50 mM sodium chloride/50 mM Tris HCl buffer solution (pH 8.5), 0.01 ml of 100 mM EDTA and 0.4 ml of Dole's reagent (i.e., isopropanol:heptane:1N sulphuric acid, 40:10:10 (V/V/V)) were added to terminate the reaction, after which 0.2 ml distilled water and 0.24 ml heptane were added and the mixture was agitated for 1 minute in a vortex mixer. Next, the mixture was centrifuged at 10,000 x g for 3 minutes and 0.2 ml of the upper layer (i.e., heptane layer) was transferred to another Eppendorf tube. Then, another 0.4 ml of heptane was added to this heptan solution, after which approximately 100 mg of powdered silica gel was added to adsorb the residual water and phospholipids in the heptane solution. Then, after mixing for 1 minute in a vortex mixer, the mixture was centrifuged at 10,000 x g for 3 minutes, and 0.4 ml of the supernatant was transferred to a liquid scintillation vial, 5 ml of scintillator was added and the radioactivity of the sample was measured; the results are shown in Table 6.

Rat pancreatic phospholipase $A_2$ (9 ng) was used as the standard, its radioactivity being compared with that of the yeast culture in order to calculate the quantity of phospholipase $A_2$ (ng/ml) expressed by the yeast. This calculation was performed with a conversion factor of 2.27 in order to take into account the difference in substrate specificity between rat and human pancreatic phospholipase $A_2$.

Table 6

| Measurements of phospholipase $A_2$ activity of secretory fraction | | | |
|---|---|---|---|
| | Phospholipase $A_2$ activity (cpm)[e) | Rat phospholipase $A_2$ (ng/ml) | Human pancreatic phospholipase $A_2$ (ng/ml) |
| Rat pancreatic phospholipase $A_2$ (9 ng) | 12001 | | |
| Yeast culture (30 $\mu\ell$; cultured for 2 days) | 600 | 15 | 34 |
| Rat pancreatic phospholipase $A_2$ (9 ng) | 14418 | | |
| Yeast culture (30 $\mu\ell$; cultured for 3 days) | 3864 | 80 | 182 |
| Rat pancreatic phospholipase $A_2$ (9 ng) | 14661 | | |
| Yeast culture (30 $\mu\ell$; cultured for 5 days) | 15105 | 309 | 703 |

e) Radioactive counts recorded during 1 minute by scintillation counter.

Table 6 shows that the production of phospholipase $A_2$ secreted into the yeast culture medium increases with incubation time, demonstrating that the phospholipase $A_2$ is indeed expressed by the yeast.

It is understood that various other modifications will be apparent to and can be readily made by those skilled in the art without departing from the scope and spirit of this invention. Accordingly, it is not intended that the scope of the claims appended hereto be limited to the description as set forth herein, but rather that the claims be construed as encompassing all the features of patentable novelty that reside in the present invention, including all features that would be treated as equivalents thereof by those skilled in the art to which this invention pertains.

**Claims**

1. A process for the production of human pancreatic phospholipase $A_2$ which comprises transforming yeast cells with a vector bearing a gene encoding human pancreatic phospholipase $A_2$ and culturing the resultant transformant under appropriate conditions.

2. A process according to claim 1, wherein the gene encodes the amino acid sequence shown in Figure 1 of the accompanying drawings or wherein the gene corresponds to the base sequence shown in Figure 1 of the accompanying drawings.

3. A process according to claim 1 or 2, wherein said vector is the plasmid pAM82 HuPLA$_2$.

4. A process according to claim 1 or 2, wherein said transformant is Saccharomyces cerevisiae pAM82-HuPLA$_2$/AH22.

5. A recombinant human pancreatic phospholipase A$_2$.

6. An expression vector for use in Saccharomyces cerevisiae carrying a gene encoding human pancreatic phospholipase A$_2$.

7. An expression vector as claimed in claim 6, being plasmid pAM82 HuPLA$_2$.

8. Saccharomyces cerevisiae transformed using an expression vector carrying a gene encoding human pancreatic phospholipase A$_2$.

9. Sacchoromyces cerevisiae pAM82-HuPLA$_2$/AH22.

10. A method for producing human pancreatic phospholipase A$_2$, comprising the steps of:
introducing a plasmid capable of expressing human pancreatic phospholipase A$_2$ into yeast cells;
culturing said yeast cells in a culture medium to induce the secretion of human pancreatic phospholipase A$_2$; and
recovering the secreted human pancreatic phospholipase A$_2$ from the culture medium.

11. A method according to claim 10, wherein said plasmid is prepared by the following steps:
isolating mRNA from human pancreas;
synthesizing double-stranded cDNA from said mRNA;
preparing a cDNA library in lambda phage from said cDNA;
screening a cDNA clone, which has a gene encoding human pancreatic phospholipase A$_2$, from said cDNA library; and
introducing the gene encoding human pancreatic phospholipase A$_2$ contained in said cDNA clone into a plasmid vector.

12. A method according to claim 10, wherein said plasmid is pAM82 HuPLA$_2$.

13. A method according to claim 12, wherein said yeast cells carrying said plasmid is Saccharomyces cerevisiae pAM82-HuPLA$_2$/AH22.

Fig. 1

TTCTTTTCTCACCTTGACTGCAAG

```
          10        20        30        40        50        60        70        80
ATGAAACTCCTTGTGCTAGCTGTGCTGCTCACAGTGGCCGCCGCCGACAGCGGCATCAGCCCTCGGGCCGTGTGGCAGTT
M  K  L  L  V  L  A  V  L  L  T  V  A  A  A  D  S  G  I  S  P  R  A  V  W  Q  F
```

```
          90       100       110       120       130       140       150       160
CCGCAAAATGATCAAGTGCGTGATCCCGGGGAGTGACCCCTTCTTGGAATACAACAACTACGGCTGCTACTGTGGCTTGG
R  K  M  I  K  C  V  I  P  G  S  D  P  F  L  E  Y  N  N  Y  G  C  Y  C  G  L  G
```

```
         170       180       190       200       210       220       230       240
GGGGCTCAGGCACCCCCGTGGATGAACTGGACAAGTGCTGCCAGACACATGACAACTGCTATGACCAGGCCAAGAAGCTG
G  S  G  T  P  V  D  E  L  D  K  C  C  Q  T  H  D  N  C  Y  D  Q  A  K  K  L
```

```
         250       260       270       280       290       300       310       320
GACAGCTGTAAATTTCTGCTGGACAACCCGTACACCCACACCTATTCATACTCGTGCTCTGGCTCGGCAATCACCTGTAG
D  S  C  K  F  L  L  D  N  P  Y  T  H  T  Y  S  Y  S  C  S  G  S  A  I  T  C  S
```

```
         330       340       350       360       370       380       390       400
CAGCAAAAACAAAGAGTGTGAGGCCTTCATTTGCAACTGCGACCGCAACGCTGCCATCTGCTTTTCAAAAGCTCCATATA
S  K  N  K  E  C  E  A  F  I  C  N  C  D  R  N  A  A  I  C  F  S  K  A  P  Y  N
```

```
         410       420       430       440
ACAAGGCACACAAGAACCTGGACACCAAGAAGTATTGTCAGAGTTGAATATCACCTCTCAAAAGCATCACCTCTATCTGC
K  A  H  K  N  L  D  T  K  K  Y  C  Q  S  *
```

CTCATCTCACACTGTACTCTCCAATAAAGCACCTTGTTGAAAGACAAAAAAAAAAAAAAAAAAAAAA

EP 0 359 425 A1

Fig. 2

| Amino acid No. | 121 | 122 | 123 | 124 | 125 | 126 |
|---|---|---|---|---|---|---|
| H P L 1 1 | K | K | Y | C | Q | S |
| V e r h e i j et al. | K | Y | S | C | Q | |

EP 0 359 425 A1

EP 0 359 425 A1

Fig. 3

Human PLA₂ (HPL11)  MKLLVLAVLLTVAAADSGISPRAVWQFRKMIKCVIPGSDPFLEYNNYGCYCGLGGSGTPVDE
Canine PLA₂         **F****A*******EG**************N****T**E***LKD**D**************


LDKCCQTHDNCYDQAKKLDSCKFLLDNPYTHTYSYSCSGSAITCSSKNKECEAFICNCD
**********H**SE******************KI*********E*********D*Q*******


RNAAICFSKAPYNKAHKNLDTKKYCQS                126
*S************E*************--             124

## Fig. 4

# EUROPEAN SEARCH REPORT

Application Number

EP 89 30 8634

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | BIOCHEMICA AND BIOPHYSICA ACTA, vol. 747, 1983, pages 93-99, Elsevier Science Publishers B.V., Amsterdam, NL; H.M. VERHEIJ et al.: "The complete primary structure of phospholipase A2 from human pancreas" * Figure * | 1-13 | C 12 N    15/55<br>C 12 N    15/81<br>C 12 N    9/18<br>C 12 N    1/18 //<br>(C 12 N    1/18<br>C 12 R    1:865) |
| Y,D | GENE, vol 64, 27th May 1988, pages 257-264, Elsevier Science Publishers B.V. (Biomedical Devision), Amsterdam NL; T. TANAKA et al.: "Secretion of the proenzyme and active bovine pancreatic phospholipase A2 enzyme by Saccharomyces cerevisiae: design and use of a synthetic gene" * Whole article * | 1-13 | |
| Y | EUROPEAN JOURNAL OF BIOCHEMISTRY, vol. 170, 1987, pages 241-246, FEBS, Berlin, DE; C.J. VAN DEN BERGH et al.: "Secretion of biologically active porcine prophospholipase A2 by Saccharomyces cerevisiae" * Whole article * | 1-13 | TECHNICAL FIELDS SEARCHED (Int. Cl.5)<br><br>C 12 N |
| A,D | DNA, vol. 5, no. 6, 1986, pages 519-527, Mary Ana Liebert, Inc., Publisher, New York, US; J.J. SEILHAMER et al.: "Pancreatic phospholipase A2: isolation of the human gene and cDNAs from porcine pancreas and human lung" | 11 | |

The present search report has been drawn up for all claims

| Place of search | Date of completion of the search | Examiner |
|---|---|---|
| THE HAGUE | 28-11-1989 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)